**Europäisches Patentamt**

**European Patent Office**

**Office européen des brevets**

(19)

(11) Veröffentlichungsnummer : **0 158 059**
**B1**

(12)                   **EUROPÄISCHE PATENTSCHRIFT**

(45) Veröffentlichungstag der Patentschrift :
30.12.86

(51) Int. Cl.⁴ : **C 07 C 85/24, C 07 C119/048**

(21) Anmeldenummer : 85101791.3

(22) Anmeldetag : 19.02.85

(54) Verfahren zur Herstellung von Polyaminen und ihre Verwendung zur Herstellung von Polyisocyanaten.

(30) Priorität : 01.03.84 DE 3407494

(43) Veröffentlichungstag der Anmeldung :
16.10.85 Patentblatt 85/42

(45) Bekanntmachung des Hinweises auf die Patenterteilung : 30.12.86 Patentblatt 86/52

(84) Benannte Vertragsstaaten :
BE DE FR GB IT NL

(56) Entgegenhaltungen :
EP-A- 0 003 303
EP-A- 0 014 927
EP-A- 0 031 423
BE-A- 737 428
US-A- 3 367 969

(73) Patentinhaber : BAYER AG
Konzernverwaltung RP Patentabteilung
D-5090 Leverkusen 1 Bayerwerk (DE)

(72) Erfinder : Keggenhoff, Berthold, Dr.
Im Paradies 32
D-4150 Krefeld 29 (DE)
Erfinder : Mählmann, Enno, Dr.
Bodelschwinghstrasse 18
D-4150 Krefeld (DE)
Erfinder : Eifler, Willi, Dr.
Katharinental 25
D-5060 Bergisch Gladbach 2 (DE)
Erfinder : Ellendt, Günther, Dr.
Deswatinesstrasse 81
D-4150 Krefeld (DE)

## Beschreibung

Die Erfindung betrifft ein neues Verfahren zur Herstellung von Polyaminen der Diphenylmethanreihe mit einem hohen Gehalt an Diaminen und einem niedrigen Gehalt an 2,2'-Diaminodiphenylmethan durch zweistufige Umsetzung von Anilin mit Formaldehyd in Gegenwart von Salzsäure als Katalysator.

Polyisocyanatgemische der Diphenylmethanreihe mit einem Diisocyanatgehalt von über 85 % haben zunehmendes Interesse gefunden, weil sie bei der Weiterverarbeitung zu Polyurethanen vielfach den technisch eingeführten hochreinen Diisocyanaten in der Qualität nur wenig nachstehen, aber als Rohisocyanate wirtschaftlich wesentlich günstiger erzeugt werden können. Für ihre Eigenschaften hat es sich vielfach als günstig erwiesen, wenn sie einen mittleren Gehalt an 2,4'-Diisocyanatodiphenylmethan von ca. 8 bis 12 % besitzen, da dadurch die Kristallisationsneigung unterdrückt wird, ohne daß die Reaktivität nennenswert beeinträchtigt wird. Die Herstellung von den diesen Isocyanaten zugrundeliegenden Polyaminen mit hohem Diaminanteil ist dem Stand der Technik im Prinzip bekannt. Sie werden erhalten, wenn die Anilin-Formaldehyd-Kondensation mit einem hohen molaren Verhältnis Anilin : Formaldehyd durchgeführt wird.

Ein wesentlicher Kostenfaktor bei der technischen Herstellung dieser Produkte ist die Einsatzmenge an Salzsäure, da diese im Normalfall durch Zusatz von Natronlauge aus dem Prozeß entfernt werden muß.

Wählt man nun einen niedrigen Protonierungsgrad (Protonierungsgrad = Prozentsatz der in Form von Ammoniumgruppen, d. h. in mit Salzsäure neutralisierter Form vorliegenden Aminogruppen, bezogen auf die Gesamtmenge aller, d. h. der neutralisierten und nicht neutralisierten Aminogruppen im Reaktionsgemisch), um trotz des hohen Anilinüberschusses, der zur Erzielung des hohen Diaminoanteils erforderlich ist, wirtschaftlich günstige spezifische Verbräuche an Salzsäure und Natronlauge zu erzielen, so erhält man nicht nur erhöhte Gehalte an 2,4'-Diaminodiphenylmethan, was oftmals erwünscht sein kann, sondern außerdem in stark zunehmendem Umfang 2,2'-Diaminodiphenylmethan. Diese Komponente führt nicht nur zu einer deutlich verminderten Reaktivität des aus den Polyaminen hergestellten Polyisocyanatgemischs, sondern auch zu einer negativen Beeinträchtigung der Eigenschaften der Polyurethan-Kunststoffe, die aus diesen Polyisocyanaten hergestellt werden.

Die US-PS 3 367 969 beschreibt ein Verfahren zur Herstellung von Polyamingemischen der Diphenylmethanreihe durch Umsetzung von 1,5 bis 6, bevorzugt 2 bis 5 Mol Anilin mit einem Mol Formaldehyd in Gegenwart von 0,25 bis 1,1, bevorzugt 0,95 bis 1,05 Mol Salzsäure je Mol Anilin bei 20 bis 50 °C, anschließende Zugabe von mindestens 0,2 Mol Anilin, so daß die Endzusammensetzung 2 bis 10, bevorzugt 2,5 bis 5 Mol Anilin pro Mol Formaldehyd und 0,1 bis 0,98 Mol, bevorzugt 0,75 bis 0,95 Mol Salzsäure pro Mol Anilin beträgt, und Erhitzen der Mischung auf 50 bis 100 °C, bis die Reaktion zu Ende geführt ist. Im Anschluß an die Neutralisation des so erhaltenen sauren Reaktionsgemisches erfolgt die Abtrennung von überschüssigem Anilin und die destillative Gewinnung des 4,4'-Diaminodiphenylmethans.

Diese Art der Herstellung von reinem 4,4'-Diaminodiphenylmethan ist jedoch mit dem Nachteil behaftet, daß erhebliche Mengen an Nebenprodukten, insbesondere in Form von höherfunktionellen Polyaminen (Destillationsrückstand) anfallen.

In den Ausführungsbeispielen des Patents wird in der ersten Stufe unter Einhaltung eines Protonierungsgrads von 100 % (geringfügiger Salzsäureüberschuß) und in der zweiten Reaktionsstufe unter Einhaltung eines Protonierungsgrads von mindestens 50 % gearbeitet. Ganz abgesehen von dem hohen Verbrauch an Salzsäure und an Natronlauge zu deren Neutralisation führt eine derartige Arbeitsweise, wie ein Nacharbeiten der Beispiele zeigt, zu einem Polyamingemisch, aus welchem zwar 4,4'-Diaminodiphenylmethan in hoher Ausbeute isoliert werden kann, welche jedoch, falls auf eine derartige destillative Aufarbeitung verzichtet wird, mit dem Nachteil behaftet ist, daß das entsprechende Phosgenierungsprodukt, d. h. das entsprechende Polyisocyanatgemisch eine starke Kristallisationsneigung bei Raumtemperatur und einen relativ hohen, die Reaktivität stark beeinträchtigenden Chlorgehalt aufweist. Schließlich besitzen die entsprechenden Polyisocyanatgemische eine dunkle Eigenfarbe.

Die der Erfindung zugrunde liegende Aufgabe bestand nun darin, ein neues Verfahren zur Herstellung von Polyaminen der Diphenylmethanreihe zur Verfügung zu stellen, welches die Herstellung von Polyaminen gestattet, die bei einem Diamingehalt von mindestens 89 Gew.-% und einem Gehalt an 2,4'-Diaminodiphenylmethan von 8 bis 12 Gew.-% die Herstellung der entsprechenden Polyisocyanatgemische gestattet, die sich durch eine hohe Reaktivität, eine geringe Kristallisationsneigung und eine helle Eigenfarbe auszeichnen. Gleichzeitig sollte das neue Verfahren unter Verwendung von vergleichsweise geringen Mengen an Salzsäurekatalysator durchführbar sein.

Diese Aufgabe konnte durch das nachstehend näher beschriebene erfindungsgemäße Verfahren gelöst werden.

Gegenstand der Erfindung ist ein Verfahren zur Herstellung von Polyaminen der Diphenylmethanreihe mit hohem Diamingehalt und niedrigem Gehalt an 2,2'-Diaminodiphenylmethan durch Kondensation von Anilin mit Formaldehyd in Gegenwart von Salzsäure als Katalysator, Neutralisation des Säurekatalysators nach Beendigung der Kondensationsreaktion und destillative Aufarbeitung des so erhaltenen Polyamingemischs, dadurch gekennzeichnet, daß man.

a) in einer ersten Reaktionsstufe 2,0 bis 3,5 Mol Anilin mit einem Mol Formaldehyd in Gegenwart von Salzsäure oder ein aus 2,0 bis 3,5 Mol Anilin und einem Mol Formaldehyd in Abwesenheit von Säurekatalysator hergestelltes Kondensationsprodukt in Gegenwart von Salzsäure unter Einhaltung eines Protonierungsgrades von 40 bis 60 % bei Temperaturen von unter 50 °C umsetzt,

b) anschließend dem Reaktionsgemisch weiteres Anilin hinzufügt, so daß das molare Verhältnis von Aminstickstoffatomen in Form von freiem Anilin und der gemäß a) gebildeten Umsetzungsprodukte zu in diesen Umsetzungsprodukten in Form von Methylenbrücken chemisch gebundenem Formaldehyd auf über 10 : 1 und bis zu 20 : 1 ansteigt und der Protonierungsgrad entsprechend abfällt,

c) das gemäß b) erhaltene Reaktionsgemisch schrittweise bis zur vollständigen Umlagerung der vorliegenden N-substituierten Zwischenprodukte in Polyamine mit unsubstituierten primären Aminogruppen erhitzt und

d) das gemäß c) erhaltene Reaktionsgemisch nach Neutralisation der Säure destillativ aufarbeitet.

Ausgangsmaterialien für das erfindungsgemäße Verfahren sind Anilin, Formaldehyd in wäßriger, üblicherweise 30- bis 50-gew.-%iger Lösung, sowie Salzsäure in üblicherweise 30- bis 37-gew.-%iger Konzentration.

In der ersten Reaktionsstufe werden die Ausgangsmaterialien in solchen Mengenverhältnissen eingesetzt, die einem Molverhältnis Anilin : Formaldehyd von 2 : 1 bis 3,5 : 1, vorzugsweise 2,5 : 1 bis 3 : 1 und einem Protonierungsgrad von 40 bis 60 % entsprechen. Die Menge des in der zweiten Reaktionsstufe zugesetzten Anilins wird dann so bemessen, daß in dem Reaktionsgemisch das Molverhältnis von Aminstickstoffatomen zu (hier bereits in Form von Methylenbrücken chemisch gebundenem) Formaldehyd über 10 : 1 und bis zu 20 : 1, vorzugsweise 11 : 1 bis 16 : 1 beträgt und der Protonierungsgrad der weiteren Anilinzugabe entsprechend reduziert ist.

Die Reaktionstemperatur liegt in der ersten Reaktionsstufe bei unter 50 °C, vorzugsweise bei 30 bis 40 °C und besonders bevorzugt bei 32 bis 38 °C. Das mit weiterem Anilin versetzte Reaktionsgemisch der ersten Reaktionsstufe wird dann in der zweiten Reaktionsstufe allmählich auf eine Endtemperatur von mindestens 100 °C, vorzugsweise mindestens 130 °C und besonders bevorzugt innerhalb des Temperaturbereichs von 130 bis 150 °C erhitzt, wobei unter einem Überdruck gearbeitet werden muß, der dem jeweiligen Dampfdruck des Gemisches zumindest entspricht.

Die Verweilzeit, bzw. bei kontinuierlicher Fahrweise die mittlere Verweilzeit des Reaktionsgemischs in der ersten Reaktionsstufe sollte mindestens 15 Min., vorzugsweise 30 bis 90 Min. betragen. Die (mittlere) Verweilzeit des Reaktionsgemischs in der zweiten Reaktionsstufe bei der genannten Endtemperatur muß ausreichend lang sein, um die quantitative Umlagerung der N-substituierten Zwischenprodukte (Aminobenzylanilin) in Polyamin mit ausschließlich primären Aminogruppen zu ermöglichen. Dies bedeutet, daß das Reaktionsgemisch solange bei der genannten Endtemperatur gehalten wird, bis sich analytisch, z. B. mit Hilfe der Hochleistungs-Flüssigkeitschromatographie, kein Aminobenzylanilin mehr nachweisen läßt.

Zu Beginn der Reaktion ist es sowohl möglich, Anilin und Salzsäure vorzumischen und danach die Formaldehydlösung zuzusetzen, als auch zunächst Anilin und Formaldehyd im genannten Molverhältnis in Abwesenheit der Säure bei beispielsweise 40 bis 100 °C zu dem entsprechenden N,N'-disubstituierten Aminal umzusetzen, gegebenenfalls das hierbei gebildete Wasser und das mit dem Formaldehyd eingebrachte Wasser durch Phasentrennung vom Kondensationsprodukt abzutrennen und das produkt dieser neutralen Vorreaktion anschließend mit der Salzsäure zu versetzen und der ersten Reaktionsstufe des erfindungsgemäßen Verfahrens zuzuführen.

Die Temperatur der Umsetzung von Formaldehyd mit der Anilin-Salzsäuremischung oder des Produkts der Anilin-Formaldehyd-Vorreaktion mit Salzsäure ist ein für die Qualität des Endproduktes wichtiger Prozeßpartner. Da diese Umsetzungen stark exotherm verlaufen, ist für gute Wärmeabfuhr zu sorgen. Bei der Durchführung des erfindungsgemäßen Verfahrens werden bevorzugt Rührkessel mit direkter Kühlung oder, entsprechend DE-PS 2 149 998, Verdampfungskühlung unter vermindertem Druck eingesetzt. Dabei wird durch entsprechende Volumenauswahl eine (mittlere) Verweilzeit von mindestens 15 Min., vorzugsweise 30 bis 90 Min., gewährleistet. Nach dieser Zeit wird das weitere Anilin hinzugefügt und die so erhaltene Mischung langsam, wie oben ausgeführt, erhitzt.

Nach Beendigung der zweiten Reaktionsstufe wird das Reaktionsgemisch in bekannter Weise, beispielsweise mit Natronlauge neutralisiert, die organische Phase von der Salzlösung getrennt, aus der organischen Phase das überschüssige Anilin abdestilliert und das erfindungsgemäße Polyamingemisch gewonnen.

Das Verfahren kann diskontinuierlich und kontinuierlich durchgeführt werden. Bei kontinuierlicher Fahrweise kann die zweite Reaktionsstufe vorteilhaft in einer Rührkesselkaskade mit anschließendem Druckreaktor oder in entsprechend dimensionierten Rohr- oder Turmreaktoren ausgeführt werden, sofern die zuvor beschriebene Temperaturführung eingehalten werden kann.

Die erhaltenen Polyamine können durch Phosgenierung zu qualitativ hochwertigen Polyisocyanaten der Diphenylmethanreihe weiterverarbeitet werden, die als solche oder nach Abmischung mit anderen Isocyanaten oder auch nach geeigneter Modifizierung als Rohstoff beispielsweise für harte und weiche Schaumstoffe sowie harte und elastische Formkörper verwendet werden können.

Die aus den erfindungsgemäßen Verfahrensprodukten hergestellten Polyisocyanate stellen wegen ihres geringen Gehalts an höherfunktionellen Polyisocyanaten aber auch ein wertvolles Ausgangsmaterial zur Herstellung von reinem 4,4'-Diisocyanatodiphenylmethan bzw. von dessen Gemischen mit 2,4'-Diisocyanatodiphenylmethan dar.

In den nachfolgenden Beispielen beziehen sich alle Prozentangaben auf Gewichtsprozente.

Beispiel 1 (Vergleich)

In einem Rührkolben werden 940 g = 10.1 Mol Anilin mit 146 g = 1,2 Mol Salzsäure 30 %ig gemischt und auf 35 °C gekühlt. Danach werden 100 g = 1 Mol wäßrige 30 %ige Formaldehydlösung unter Rühren zugegeben, wobei durch Kühlung die Temperatur der Mischung bei 35 °C gehalten wird. Die Mischung wird insgesamt 60 Min. bei 35 °C gehalten, dann 60 Min. bei 45 °C gerührt und in weiteren 60 Min. auf 100 °C erhitzt. Diese Mischung wird in einem Druckbehälter überführt, auf 135 °C erhitzt und 60 Min. bei dieser Temperatur gehalten. Nach Abkühlung wird die Mischung mit 120 g = 1,5 Mol Natronlauge 50 %ige neutralisiert, die entstehende Salzlösung durch Phasenscheidung abgetrennt und von der organischen Phase Wasser und freies Anilin abdestilliert. Die Ausbeute an Polyamingemisch beträgt 191 g. Daraus berechnet sich der spezifische Salzsäureverbrauch zu 229 g HCl 100 %/kg Polyamin.

Das Produkt hat die folgende Zusammensetzung (MDA = Abkürzung für Diaminodiphenylmethan) :

75,2 % 4,4'-MDA
13,8 % 2,4'-MDA
1,0 % 2,2'-MDA
9,9 % Polyamine

Beispiel 2 (Vergleich entsprechend Beispiel 1, Tafel I, Run 6 der US-PS 3 367 969)

In einem Rührkolben werden 279 g = 3 Mol Anilin mit 369 g = 3,03 Mol Salzsäure 30 %ig gemischt. Danach werden bei 40 °C 100 g = 1 Mol wäßrige 30 %ige Formaldehydlösung unter Rühren zugegeben. Die Temperatur wird 30 Min. gehalten. Dann werden 284,6 g = 3,06 Mol Anilin zugegeben, die Temperatur auf 65 °C erhöht und die Mischung drei Stunden bei dieser Temperatur gehalten. Die Reaktionsmischung wird mit 304 g = 3,8 Mol Natronlauge 50 %ig neutralisiert, die entstehende Salzlösung durch Phasenscheidung abgetrennt und von der organischen Phase Wasser und freies Anilin abdestilliert. Die Ausbeute an Polyamingemisch beträgt 191 g. Daraus berechnet sich der spezifische Salzsäureverbrauch zu 580 g HCl 100 %/kg Polyamin.

Das produkt hat die folgende Zusammensetzung :

85,2 % 4,4'-MDA

5,0 % 2,4'-MDA
0,1 % 2,2'-MDA
9,8 % Polyamin.

Zur Gewinnung des Polyisocyanats werden 100 g Polyamin in 510 ml Chlorbenzol gelöst. In einem Rührkolben wird eine Lösung von 150 ml flüssigem Phosgen in 625 ml Chlorbenzol hergestellt. Unter Rühren wird die Polyaminlösung hinzugegeben und die erhaltene Mischung innerhalb 45 Min. auf 130 °C erhitzt, wobei Chlorwasserstoff mit Phosgen gemischt als Abgas abgeführt werden.

Die so erhaltene Lösung wird durch Destillation von Phosgen und Chlorbenzol befreit, wobei die Endtemperatur des Produkts 240 °C beträgt. Anschließend wird das gewonnene Polyisocyanatgemisch durch die folgenden Angaben charakterisiert :

Chlorgehalt (%) : 0,36
Extinktion einer 5 %igen Lösung in Chlorbenzol bei 430 nm : 0,348
Kristallisationsverhalten bei 25 °C : kristallisiert innerhalb 8 Std.

Beispiel 3 (erfindungsgemäß)

In einem Rührkolben werden 260 g = 2,8 Mol Anilin mit 146 g = 1,2 Mol Salzsäure 30 %ig gemischt und auf 35 °C gekühlt. Danach werden 100 g = 1 Mol wäßrige 30 %ige Formaldehydlösung unter Rühren zugegeben, wobei durch Kühlung die Temperatur der Mischung bei 35 °C gehalten wird. Die Mischung wird insgesamt 60 Min. bei 35 °C gerührt. Dann werden 680 g = 7,3 Mol Anilin zugegeben, die Mischung auf 45 °C erwärmt, 60 Min. bei 45 °C gerührt und in weiteren 60 Min. auf 100 °C erhitzt. Diese Mischung wird in einen Druckbehälter überführt, auf 135 °C erhitzt und 60 Min. bei dieser Temperatur gehalten. Nach Abkühlung wird die Mischung mit 120 g = 1,5 Mol Natronlauge 50 %ig neutralisiert, die entstehende Salzlösung durch Phasenscheidung abgetrennt und von der organischen Phase Wasser und freies Anilin abdestilliert. Die Ausbeute an Polyamingemisch beträgt 191 g. Daraus berechnet sich der spezifische Salzsäureverbrauch zu 229 g HCl 100 %/kg Polyamin.

Das Produkt hat die folgende Zusammensetzung :

79,7 % 4,4'-MDA
9,6 % 2,4'-MDA
0,5 % 2,2'-MDA
10,1 % Polyamin.

Das Polyamingemisch wird in genau gleicher Weise wie im Beispiel 2 mit Phosgen zum Isocyanat umgesetzt. Das erhaltene Polyisocyanatgemisch wird durch die folgenden Angaben charakterisiert :

Chlorgehalt (%) : 0,20
Extinktion einer 5 %igen Lösung in Chlorbenzol bei 430 nm : 0,232
Kristallisationsverhalten bei 25 °C : nach 8 Tagen keine Kristallisation

Beispeil 4 (erfindungsgemäß)

In einem Rührkolben werden 260 g = 2,8 Mol Anilin bei 50 °C mit 100 g = 1 Mol Formaldehyd in Form einer 30 %igen wäßrigen Lösung säurefrei umgesetzt. Das zweiphasige Gemisch trennt sich beim Abstellen des Rührers in eine obere wäßrige und eine untere organische Phase.

Die wäßrige Phase wird abgetrennt und die organische Phase wird mit 146 g = 1,2 Mol 30 %iger Salzsäure unter Rühren gemischt, wobei die Temperatur bei 35 °C gehalten wird. Die Mischung wird 60 Min. bei 35 °C gehalten, dann werden 857 g = 9,2 Mol Anilin hinzugegeben, die Lösung auf 45 °C erwärmt und 60 Min. auf 100 °C erhitzt, anschließend in einen Druckbehälter überführt und in diesem auf 140 °C erhitzt und 20 Min. bei dieser Temperatur gehalten. Nach dem Abkühlen wird die Mischung mit 120 g = 1,5 Mol Natronlauge 50 %ig neutralisiert, die entstehende Salzlösung durch Phasenscheidung abgetrennt und von der organischen Phase Wasser und freies Anilin abdestilliert. Die Ausbeute an Polyamingemisch beträgt 191 g. Der spezifische Salzsäureverbrauch beträgt 229 g HCl 100 %/kg Polyamin.

Das Produkt hat die folgende Zusammensetzung :

| | |
|---|---|
| 79,5 % | 4,4'-MDA |
| 10,8 % | 2,4'-MDA |
| 0,35 % | 2,2'-MDA |
| 9,35 % | Polyamin. |

Beispiel 5 (Vergleich)

Die Durchführung entspricht Beispiel 1 mit folgenden Mengen :

| | |
|---|---|
| Anilin | 15 Mol = 1 395 g |
| Salzsäure | 1,2 Mol = 146 g |
| Formaldehyd (30 %ige wäßrige Lösung) | 1 Mol = 100 g |
| Natronlauge | 1,5 Mol = 120 g |

Das Polyamingemisch hat die folgende Zusammensetzung :

76,3 % 4,4'-MDA
15,9 % 2,4'-MDA
1,3 % 2,2'-MDA
6,5 % Polyamine.

Beispiel 6 (erfindungsgemäßes kontinuierliches Verhalten)

Eine kontinuierliche Versuchsanlage besteht aus 6 als Kaskade geschalteten Rührkolben von 2 l Inhalt und einem nachgeschalteten, unter 5 bar Überdruck betriebenen Rohrreaktor von 4 l Inhalt. Der erste Rührkolben wird unter einem Unterdruck von 40 mbar bei Rückfluß betrieben, so daß er eine Mediumstemperatur von 34 °C aufweist.

Der zweite Rührkolben wird bei 45 °C, der dritte bei 50 °C, der vierte bei 60 °C, der fünfte bei 75 °C und der sechste bei 95 °C betrieben. Aus dem sechsten Rührkolben wird die Mischung mittels Pumpe in den Rohrreaktor gefördert, bei dem am Anfang eine Mediumstemperatur von 100 °C eingestellt wird, die stetig ansteigt und am Ablauf 140 °C beträgt. Die ablaufende saure Lösung wird abgekühlt und gesammelt.

In der ersten Kolben werden stündlich 1 600 g eine Mischung aus 1 025 g Anilin und 575 g Salzsäure 30 %ig sowie zusätzlich 394 g 30 %ige wäßrige Formaldehydlösung eingespeist. Dies entspricht einem Molverhältnis Anilin : Formaldehyd von 2,8 : 1 und einem Protonierungsgrad von 43 %. In diese Mischung wird beim Überlaufen in den zweiten Kolben stündlich 4 471 g Anilin zugegeben. Dies entspricht einem Molverhältnis von Aminstickstoffatomen zu chemisch gebundenem Formaldehyd 15 : 1. Freier Formaldehyd liegt hier nicht mehr vor. 6 465 g der am Rohrreaktor ablaufenden Mischung werden durch Zusatz von 525 g Natronlauge 50 %ig neutralisiert, die entstehende Salzlösung durch Phasenscheidung abgetrennt und aus der organischen Phase Wasser und freies Anilin abdestilliert. Die stündliche Ausbeute an Polyamingemisch beträgt 750 g. Der spezifische Säureverbrauch berechnet sich zu 230 g HCl 100 %/kg Polyamin.

Das Produkt hat die folgende Zusammensetzung :

| | |
|---|---|
| 4,4'-MDA | 81,8 % |
| 2,4'-MDA | 9,3 % |
| 2,2'-MDA | 0,4 % |
| Polyamine | 8,5 % |

Das Polyamingemisch wird wie im Beispiel 2 mit Phosgen zum Isocyanat umgesetzt. Das erhaltene Polyisocyanatgemisch wird durch die folgenden Angaben charakterisiert :
Chlorgehalt (%) : 0,11
Extinktion einer 5 %igen Lösung in Chlorbenzol bei 430 nm : 0,158
Kristallisationsverhalten bei 25 °C : nach 8 Tagen keine Kristallisation.

**Patentansprüche**

1. Verfahren zur Herstellung von Polyaminen der Diphenylmethanreihe mit hohem Diamingehalt und niedrigem Gehalt an 2,2'-Diaminodiphenylmethan durch Kondensation von Anilin mit Formaldehyd in Gegenwart von Salzsäure als Katalysator, Neutralisation des Säurekatalysators nach Beendigung der Kondensationsreaktion und destillative Aufarbeitung des so erhaltenen Polyamingemischs, dadurch gekennzeichnet, daß man

a) in einer ersten Reaktionsstufe 2,0 bis 3,5 Mol Anilin mit einem Mol Formaldehyd in Gegenwart von Salzsäure oder ein aus 2,0 bis 3,5 Mol Anilin und einem Mol Formaldehyd in Abwesenheit von Säurekatalysator hergestellten Kondensationsprodukt in Gegenwart von Salzsäure unter Einhaltung eines Protonierungsgrades von 40 bis 60 % bei Temperaturen von unter 50 °C umsetzt,

b) anschließend dem reaktionsgemisch weiteres Anilin hinzufügt, so daß das molare Verhältnis von Aminstickstoffatomen in Form von freiem Anilin und der gemäß a) gebildeten Umsetzungsprodukte zu in diesen Umsetzungsprodukten in Form von Methylenbrücken chemisch gebundenem Formaldehyd auf über 10 : 1 und bis zu 20 : 1 ansteigt und der Protonierungsgrad entsprechend abfällt,

c) das gemäß b) erhaltene Reaktionsgemisch schrittweise bis zur vollständigen Umlagerung der vorliegenden N-substituierten Zwischenprodukte in Polyamine mit unsubstituierten primären Aminogruppen erhitzt und

d) das gemäß c) erhaltene Reaktionsgemisch nach Neutralisation der Säure destillativ aufarbeitet.

2. Verfahren nach Anspruch 1, dadurch gekennzeichnet, daß man die Stufe a) bei 30 bis 40 °C durchführt.

3. Verfahren nach Anspruch 1 und 2, dadurch gekennzeichnet, daß die Verweilzeit des Reaktionsgemischs in der Stufe a) mindestens 15 Min. beträgt.

4. Verfahren gemäß Anspruch 1 bis 3, dadurch gekennzeichnet, daß man das in Stufe b) zugesetzte Anilin so bemißt, daß das Molverhältnis von Aminstickstoffatomen zu gebundenem Formaldehyd von 11 : 1 bis 16 : 1 beträgt.

5. Verfahren nach Anspruch 1 bis 4, dadurch gekennzeichnet, daß man das Reaktionsgemisch in der Stufe c) unter Druck auf Temperaturen von mindestens 130 °C erhitzt.

6. Verwendung der nach Anspruch 1 bis 6 erhaltenen Polyamingemische als Ausgangsmaterial zur Herstellung von Polyisocyanaten.

## Claims

1. Process for the production of polyamines of the diphenylmethane series having a high diamine content and a low 2,2'-diaminodiphenylmethane content by condensing aniline with formaldehyde in the presence of hydrochloric acid as catalyst, neutralizing the acid catalyst at the end of the condensation reaction and working up the polyamine mixture thus obtained by distillation, characterized in that

a) in a first reaction stage, 2.0 to 3.5 moles of aniline are reacted with one mole of formaldehyde in the presence of hydrochloric acid or a condensation product produced from 2.0 to 3.5 moles of aniline and one mole of formaldehyde in the absence of acid catalyst is reacted in the presence of hydrochloric acid while maintaining a degree of protonation of 40 to 60 % at temperatures below 50 °C,

b) more aniline is then added to the reaction mixture so that the molar ratio of amine nitrogen atoms in the form of free aniline and the reaction products formed in accordance with a) to formaldehyde chemically bound in the form of methylene bridges in these reaction products rises to beyond 10 : 1 and up to 20 : 1 and the degree of protonation decreases correspondingly,

c) the reaction mixture obtained in accordance with b) is heated in steps until the N-substituted intermediate products present have been completely rearranged into polyamines containing unsubstituted primary amino groups and

d) the reaction mixture obtained in accordance with c) is worked up by distillation after neutralization of the acid.

2. Process according to Claim 1, characterized in that stage a) is carried out at 30 to 40 °C.

3. Process according to Claim 1 and 2, characterized in that the residence time of the reaction mixture in stage a) amounts to at least 15 minutes.

4. Process according to Claim 1 to 3, characterized in that the quantity of aniline added in stage b) is measured in such a way that the molar ratio of amine nitrogen atoms to bound formaldehyde amounts to between 11 : 1 and 16 : 1.

5. Process according to Claim 1 to 4, characterized in that, in stage c), the reaction mixture is heated under pressure to temperatures of at least 130 °C.

6. Use of the polyamine mixtures obtained in accordance with Claim 1 to 6 as starting material for the production of polyisocyanates.

## Revendications

1. Procédé de fabrication des polyamines de la série du diphénylméthane ayant une teneur élevée en diamines et une basse teneur en 2,2'-diaminodiphénylméthane, par condensation de l'aniline avec de la formaldéhyde en présence d'acide chlorhydrique comme catalyseur, par neutralisation du catalyseur acide au terme de la réaction de condensation et par traitement distillatoire du mélange de polyamines obtenu, caractérisé en ce que

a) dans un premier stade de réaction on fait réagir 2,0 à 3,5 moles d'aniline avec une mole de formaldéhyde en présence d'acide chlorhydrique, ou un produit de condensation préparé à partir de 2,0 à 3,5 moles d'aniline et d'une mole de formaldéhyde en l'absence de catalyseur acide, en présence d'acide chlorhydrique, avec maintien d'un degré de protonisation de 40 à 60 % à des températures inférieures à 50 °C,

b) on ajoute ensuite au mélange de réaction un supplément d'aniline pour que le rapport molaire des atomes d'azote aminés sous forme d'aniline libre et des produits de réaction formés selon a) envers la formaldéhyde liée chimiquement dans ces produits de réaction sous la forme de ponts méthylène, s'élève à plus de 10 : 1 et jusqu'à 20 : 1 et pour que le degré de protonisation chute en rapport,

c) on chauffe le mélange de réaction obtenu selon b) progressivement jusqu'à transposition totale des produits intermédiaires N-substitués présents en polyamines avec groupes amino primaires non-substitués et

d) après neutralisation de l'acide on traite le mélange de réaction obtenu en c) par distillation.

2. Procédé selon la revendication 1, caractérisé en ce qu'on exécute le stade a) à 30-40 °C.

3. Procédé selon les revendications 1 et 2, caractérisé en ce que le temps de séjour du mélange de réaction dans le stade a) s'élève au moins à 15 minutes.

4. Procédé selon les revendications 1 à 3, caractérisé en ce qu'on règle l'aniline ajoutée au stade b) pour que le rapport molaire des atomes d'azote aminés envers la formaldéhyde liée soit de 11 : 1 à 16 : 1.

5. Procédé selon les revendications 1 à 4, caractérisé en ce qu'on chauffe le mélange de réaction dans le stade c) sous pression à des températures d'au moins 130 °C;

6. Utilisation des mélanges de polyamines obtenus selon les revendications 1 à 6 comme matière première pour la fabrication de polyisocyanates.